# EUROPEAN PATENT APPLICATION

(11) **EP 3 123 882 A1**
(43) Date of publication of application: **01.02.2017**
(21) Application number: 15178858.5
(22) Date of filing: 29.07.2015
(51) Int. Cl.: A41D 13/12, A61B 5/00, A61B 5/11, A61B 5/01, A61B 5/024

(54) **STRUCTURE OF DETECTIVE GARMENT**

(71) Applicant: King's Metal Fiber Technologies Co., Ltd., 42060 Taichung City (TW)
(72) Inventor: HOU, Jaang Jiun, Taipei City 10451 (TW); WANG, Hao Chen, Taipei City 10451 (TW); CHANG, Li Chuan, Taipei City 10451 (TW); LIAO, Shu Fen, Taipei City 10451 (TW); CHEN, Reng Sho, Taipei City 10451 (TW)
(74) Representative: Cabinet Chaillot

(57) **Abstract**

A structure of a detective garment includes a garment body and at least one detection module combined with the garment body. The garment body, when worn on a human body, detects a variation of a physiological signal of the human body or a variation of an inclination angle of the human body. The garment body includes at least one opening section in one side thereof to facilitate wearing of the garment body with one hand.

## Description

### BACKGROUND OF THE INVENTION

### 1. Field of the Invention

The present invention relates to a structure of a detective garment, and more particularly to a structure that comprises at least one opening section formed in a portion of a garment body to provide an advantage of being easily wearable and to be suitable for applications in a physiological detective garment, an inclination detective garment, or any clothing or garment-like article that comprises a detection module combined therewith.

### 2. Description of Related Art

When the heart of a person is uncomfortable, a medical doctor may ask the person to carry a heartbeat detection device to monitor and record heartbeats for one day or an even longer period of time in order to provide the doctor with the record of the heartbeats for determining if there is any abnormal situation.

However, to properly work, the heartbeat detection device must be kept in contact with a surface layer of the skin of the patient and consequently, the heartbeat detection device must be fixed on the patient's body. This often results in discomfort of the patient and even refusal of using the device.

Recently, the progress of science and technology bring a solution of combining the detection devices with clothing for facilitating monitoring and recording of the physiological conditions of a patient. This can be used for self-management of home healthcare and preventive healthcare.

However, for self-management of home healthcare in the home environment, persons that are disabled for limb movement, the elders, or surgical operation patients would encounter extreme difficulties to wear a garment that includes a detection device attached thereto due to stiffness or injury of their bodies. It also becomes sufferings for the limb-disabled persons, the elders, and the surgical operation patients to later take off a garment that includes a detection device attached thereto. Under this condition, the detection device may get readily damaged during putting on and taking off the garment, or the garment gets torn. Assistance from other persons, such as family members or attendants, is generally necessary for putting on or taking off such a garment that includes the detection device attached thereto.

Thus, in view of the above problems, the present invention aims to provide a structure of a detective garment that allows for easy wearing and thus provides conveniences for use.

### SUMMARY OF THE INVENTION

The primary objective of the present invention is to provide a structure of a detective garment, comprises a garment body and at least one detection module combined with the garment body. The garment body, when worn on a human body, detects a variation of a physiological signal of the human body or a variation of an inclination angle of the human body. The garment body comprises at least one opening section in one side thereof to facilitate wearing of the garment body with one hand thereby achieving improvement of utilization thereof.

A secondary objective of the present invention is to provide a structure of a detective garment, which comprises a garment body that can be a single-shoulder type garment, a dual-shoulder type garment, or a garment of other types. When the garment is of the single-shoulder type, the garment body comprises an opening section that is provided with a fastening section. The fastening section can be arranged at a stitching site of one lateral side of the garment body or at a left side, a right side, a front side, a rear side, a middle portion, a top portion, or any other locations of the garment body. When the garment is of the dual-shoulder type, the garment body comprises an opening section that is arranged at a shoulder and a lateral side of the garment. The opening section comprises a fastening section for each of the shoulder and the lateral side. The fastening section of the lateral side can be arranged at a stitching site of the lateral side of the garment body or at a left side, a right side, a front side, a rear side, a middle portion, a top portion, or any other locations of the garment body. The fastening section of the single-shoulder type garment or the dual-shoulder type garment can be one of a zipper, hook-and-loop fasteners, a button assembly, a hook assembly as a structure for closing or fastening thereby improving convenience thereof.

Another objective of the present invention is to provide a structure of a detective garment, which comprises a garment body with which a detection module is combined, wherein the detection module comprises an electrode plate that detects a physiological signal of a surface layer of a human body. The physiological signal can be one of body temperature, heartbeat, and pulse. The detection module may alternatively comprise an inclination detection chip and a microcontroller. The microcontroller is connected to the inclination detection chip so that the microcontroller may detect a variation of the inclination detection chip, whereby in addition to detection of a variation of a physiological signal of a human body, the detection module may also detect a variation of an inclination angle of the human body in order to identify any event of dizziness or fall to thereby improve security and safety of the user.

To achieve the above objectives, the present invention provides a structure of a detective garment, which comprises a garment body and at least one detection module mounted to the garment body. The garment body comprises at least one opening section arranged at one side or a portion thereof.

### BRIEF DESCRIPTION OF THE DRAWINGS

The present invention can be fully understood from the following detailed description and preferred embodiment with reference to the accompanying drawings, in which:
Figure 1 is a perspective view shows a dual-shoulder type garment with a fastening section of a first example according to the present invention;
Figure 2 is a perspective view shows the dual-shoulder type garment with the fastening section of the first example, in an opened condition, according to the present invention;
Figure 3 is a perspective view shows a single-shoulder type garment with a fastening section of a first example according to the present invention;
Figure 4 is a perspective view shows the single-shoulder type garment with the fastening section of the first example, in an opened condition, according to the present invention;
Figure 5 is a perspective view shows a dual-shoulder type garment with a fastening section of a second example according to the present invention;
Figure 6 is a perspective view shows the dual-shoulder type garment with the fastening section of the second example, in an opened condition, according to the present invention;
Figure 7 is a perspective view shows a dual-shoulder type garment with a fastening section of a third example according to the present invention; and
Figure 8 is a perspective view shows the dual-shoulder type garment with the fastening section of the third example, in an opened condition, according to the present invention.

### DETAILED DESCRIPTION OF THE PREFERRED EMBODIMENTS

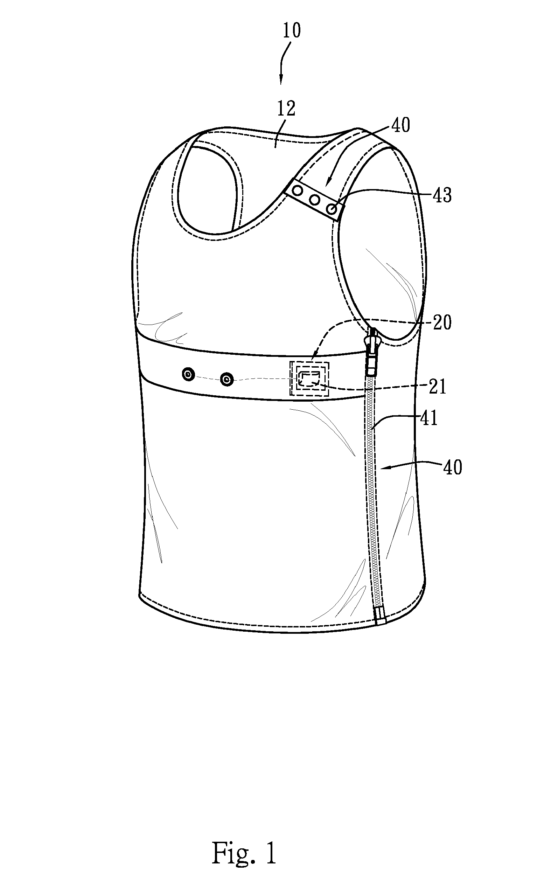

Referring to Figures 1-8, which are views illustrating embodiments of the present invention, a preferred embodiment of a structure of a detective garment according to the present invention is applicable in self-management of home healthcare for being worn on limb-disabled persons, the elder, and surgical operation patients by allowing the limb-disabled persons, the elder, and the surgical operation patients to wear with one single hand and to monitor a physiological signals or variation of a body inclination angle of the limb-disabled persons, the elder, and the surgical operation patients in order to acquire a physiological signal of the user and to prevent unexpected accidents of the limb-disabled persons, the elder, and the surgical operation patients.

The structure of the detective garment according to the present invention comprises a garment body 10 and at least one detection module 20 mounted to the garment body 10. A first structure of the detection module 20 comprises an electrode plate 21

(as shown in Figures 1-8) that detects a physiological signal of a surface layer of a human body. The electrode plate 21 is formed by weaving, knitting, or otherwise combining a plurality of non-conductive fiber yarns and a plurality of conductive fiber yarns; or alternatively, the electrode plate 21, in the entirety thereof, is formed solely of a plurality of conductive fiber yarns through weaving, knitting, or other combining means so as to make the entirety of the electrode plate 21 electrically conductive. Further, the plurality of conductive fiber yarns of the electrode plate 21 are woven, knitted, or otherwise combined to form a conductive zone. The conductive zone of the electrode plate 21 is positionable against and thus in tight contact engagement with the skin of the human body in order to detect the physiological signal. The physiological signal can be one of body temperature, heartbeat, and pulse.

A second structure of the detection module 20 comprises an inclination detection chip and a microcontroller (not shown). The microcontroller is connected to the inclination detection chip so that the microcontroller may detect a variation of the inclination detection chip. The inclination detection chip can be a three-axis acceleration transducer (such as a three-axis low-g micro-machined accelerometer). The three-axis acceleration transducer is operable to detect/calculate inclination angles and accelerations in three axes of X, Y, and Z and the microcontroller is operable to periodically detect and transmit these values. The inclination angle can be used to detect an event of dizziness or fall by setting a change exceeding a threshold of +/-1.0 degree, +/-1.5 degrees, or +/-2.0 degrees. The microcontroller can be set to conduct detection at a fixed time interval with a fixed number of detection operations. For example, abnormal inclination may be identified by means of an average of successively detected values within a period of 30 seconds being determined exceeding +/-1.0 degree (or +/-1.5 degrees or +/-2.0 degrees). Or alternatively, a nine-axis body position transducer may be used. The nine-axis body position transducer comprises a three-axis acceleration sensor, three-axis magnetic field sensor, and a three-axis gyro sensor with the range of magnetic field being ±1.3/1.9/2.5/4.0/4.7/5.6/8.1 gausses, the range of the acceleration being ±2g/±4g/±8g, and the range of the gyro being ±250/500/2000 dps. Abnormality can be identified by determining by monitoring and detecting a value of a body position according to the above-mentioned ranges. When the detected value exceeds the ranges, abnormal inclination is identified. When a variation of the inclination or body position exceeds a setting value or a threshold, the microcontroller issues, through a wireless signal transmitter (not shown), a signal.

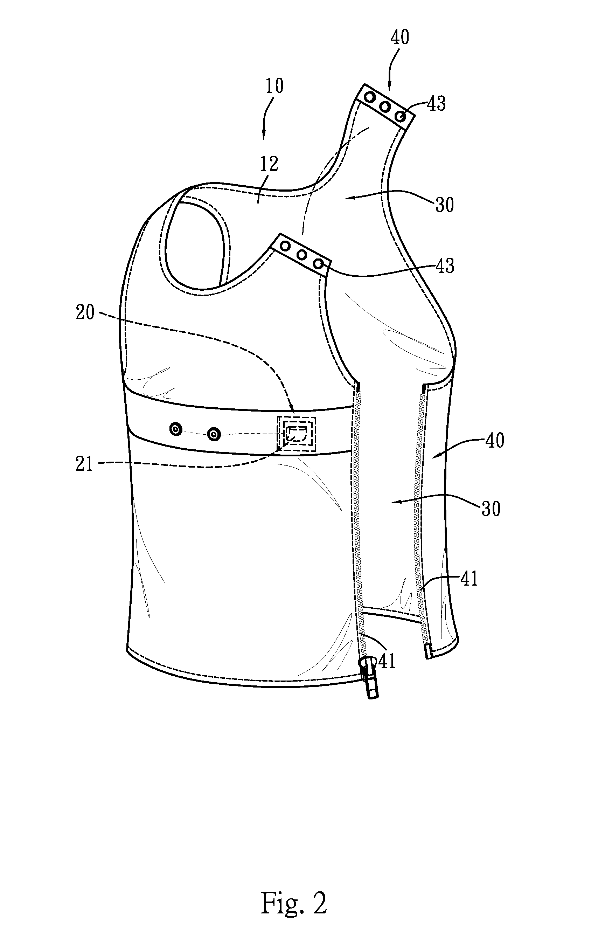

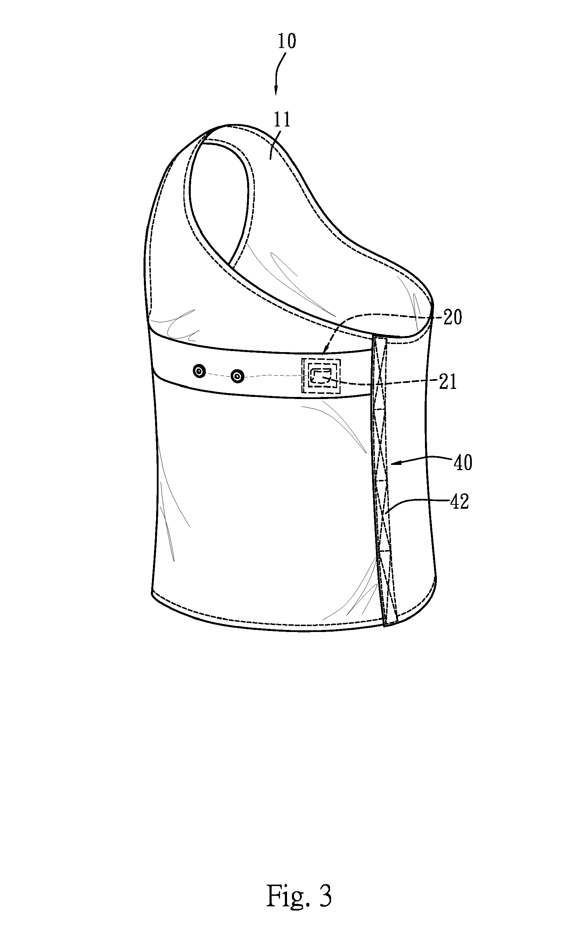

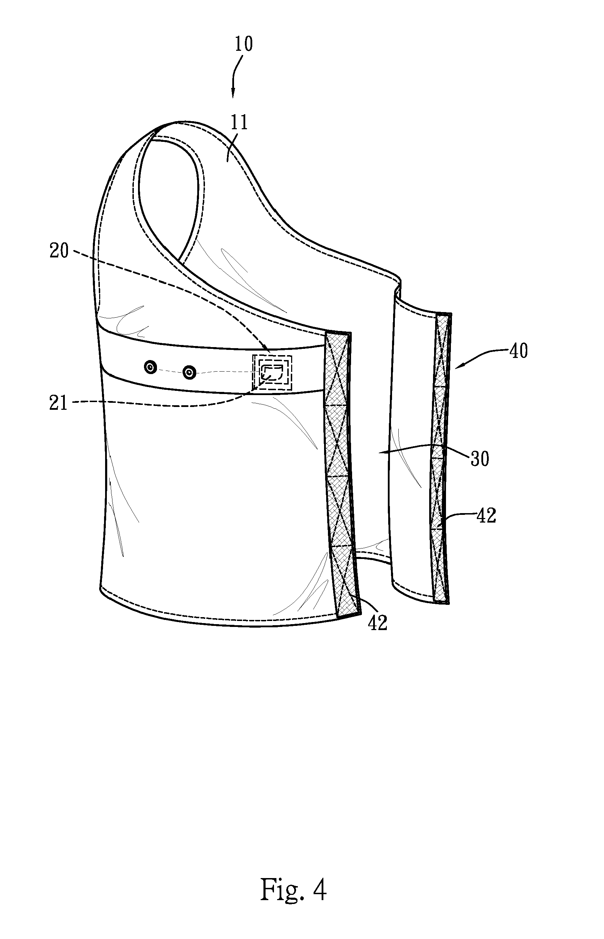

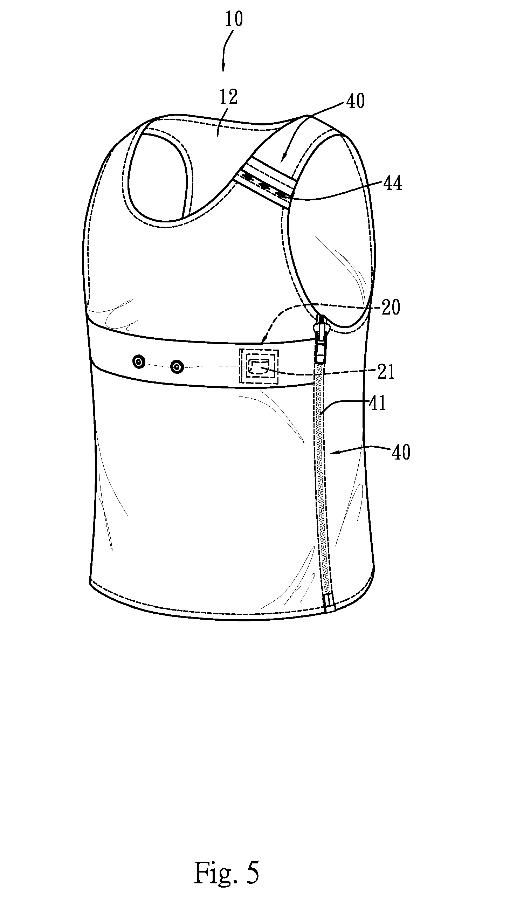

Further, the garment body 10 is formed by weaving, knitting or otherwise combining yarns. The garment body 10 can be a single-shoulder type garment 11 (such as a sleeve-less single-shoulder vest or a single-shoulder tube-top underwear) (as shown in Figures 3 and 4) or a dual-shoulder type garment 12 (such as a sleeve-less dual-shoulder vest or a thin shoulder strip vest) (as shown in Figures 5-8) or garments of other types (such as long sleeve dresses and short sleeve dresses). The garment body 10 comprises at least one opening section 30 formed at one side or in a portion thereof (as shown in Figure 2). The opening section 30 of the garment body 10 is provided with a fastening section 40. The side of the garment where the opening section 30 is formed can be a left side, a right side, a front side, a rear side, a middle portion, a top portion, or any other location of the garment body 10 and the present invention is not limited to what illustrated in the drawings. Further, the opening section 30 of the present invention can be arranged at a sewing site of the garment body 10 (as shown in Figure 2) to prevent undesired loosening and release of yarns.

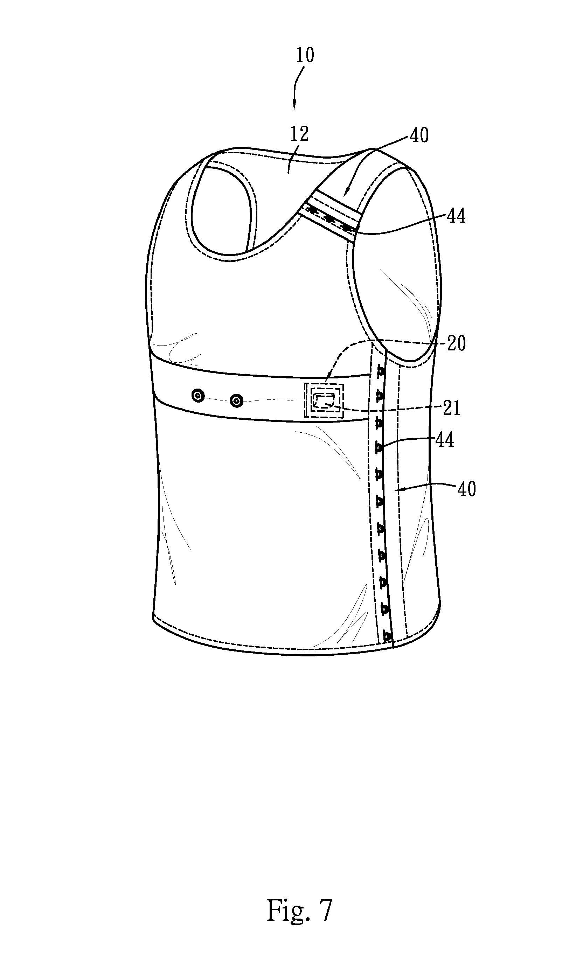

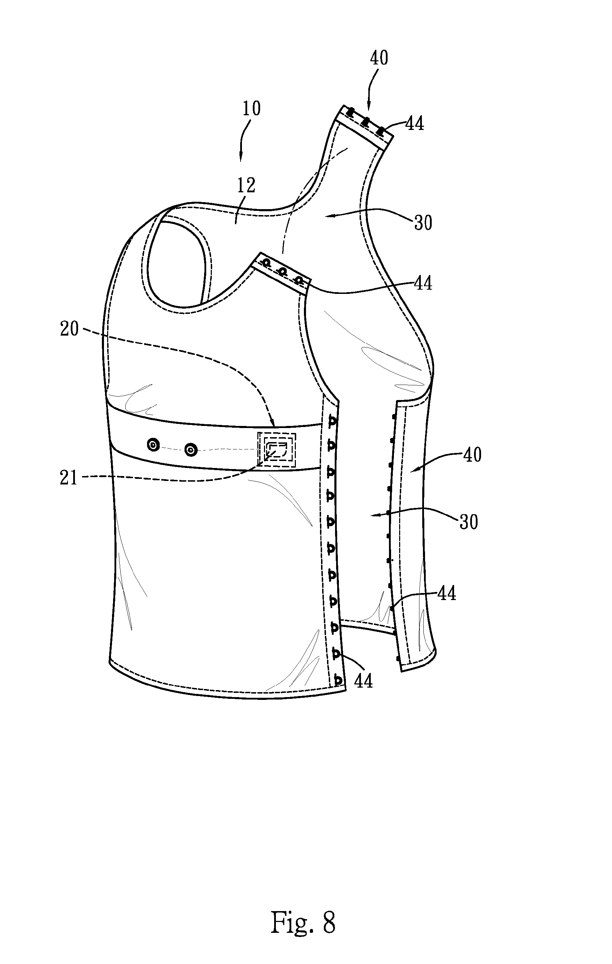

If the garment body 10 is a single-shoulder type 11, the opening section 30 of the garment body 10 comprises a fastening section 40 (as shown in Figure 3) and the fastening section 40 is arranged at a stitching site at a lateral side of the garment body 10 so that the fastening section 40 can be securely stitched to the stitching site to prevent easy detachment of the fastening section 40. Further, the fastening section 40 may also be arranged at a left side, a right side, a front side, a rear side, a middle portion, a top portion, or any other location of the garment body 10 and the fastening section 40 may comprise hook-and-loop fasteners 42 (as shown in Figures 3 and 4), other examples of the fastening section 40 including a zipper 41 (see embodiment of Figure 1), button assemblies 43 (see embodiment of Figure 2), hook assemblies 44 (see embodiment of Figure 7). The button assembly 43 can be snaps, buttons, magnetic buttons, press-fitting buttons, male-female buttons, snap-on buttons, coupling buttons, and fastening buttons and arrangements of the button assembly 43 can be one of single snap/button, dual snaps/buttons, arrayed snaps/buttons (see embodiment of Figure 2). The hook assembly 44 can be composed of mating parts of a fabric eyelet and a hook, or an eye and a hook, or two V-shaped hooks, or any other types of hook assembly 44. The arrangement of the hook assembly 44 can be one of single hook, dual hook, or arrayed hooks (see embodiment of Figure 8).

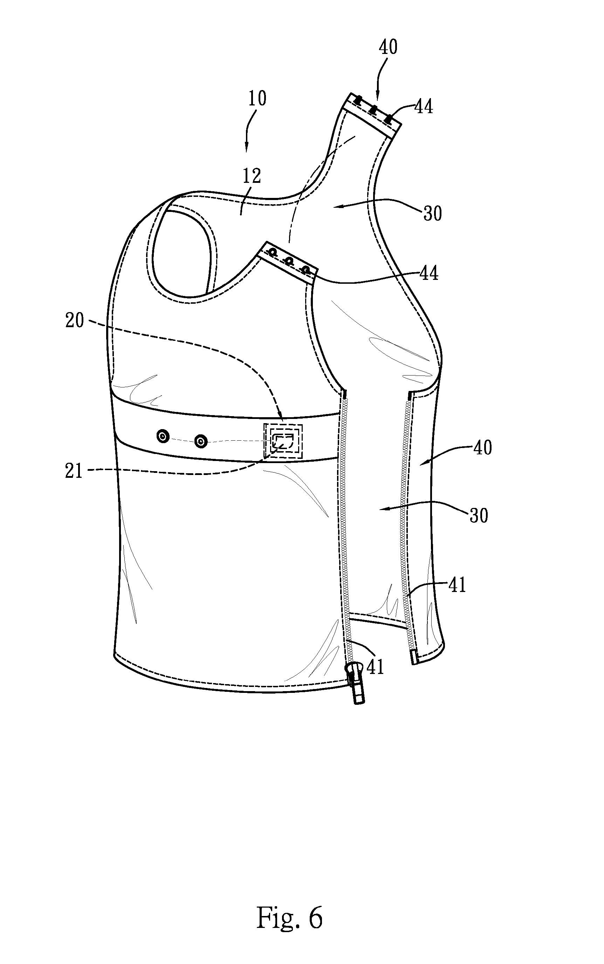

If the garment body 10 is a dual-shoulder type 12, the opening section 30 of the garment body 10 is arranged at both a shoulder and a lateral side thereof. The opening section 30 that is arranged at a shoulder and a lateral side comprises, for each of the shoulder and the lateral side, a fastening section 40 (as shown in Figures 1 and 2), wherein the fastening section 40 of the lateral side is arranged on a stitching site of the lateral side of the garment body 10 to allow the fastening section 40 to be securely stitched to the stitching site to prevent easy detachment of the fastening section 40. Further, the fastening section 40 may also be arranged at a left side, a right side, a front side, a rear side, a middle portion, a top portion, or any other location of the garment body 10 and the fastening section 40 may comprise one of a zipper 41 (as shown in Figures 5 and 6), a button assembly 43 (as shown in Figures 1 and 2), a hook assembly 44 (as shown in Figures 7 and 8), other examples of the fastening section 40 including hook-and-loop fasteners 42 (see embodiment of Figure 3) or other different types of fasteners. The button assembly 43 can be snaps, buttons, magnetic buttons, press-fitting buttons, male-female buttons, snap-on buttons, coupling buttons, and fastening buttons and arrangements of the button assembly 43 can be one of single snap/button, dual snaps/buttons, arrayed snaps/buttons (see embodiment of Figure 1). The hook assembly 44 can be composed of mating parts of a fabric eyelet and a hook, or an eye and a hook, or two V-shaped hooks, or any other types of hook assembly 44. The arrangement of the hook assembly 44 can be one of single hook, dual hook, or arrayed hooks (see embodiment of Figure 8).

To use, the fastening section 40 improves convenience and easiness of wearing the garment body 10 through the opening section 30. The arrangement of the fastening section 40 allows the limb-disabled persons, the elder, and the surgical operation patients to put on and take off the garment body 10 with one single hand so as to facilitate the convenience thereof.

Various embodiments and examples have been presented above to facilitate wearing by the limb-disabled persons, the elder, and the surgical operation patients, and in an actual use, it only needs to combine at least one detection module 20 with the garment body 10 and to provide at least one opening section 30 at one side or portion of the garment body 10 to allow for monitoring and detecting a physiological signal or a variation of a body inclination angle of the limb-disabled persons, the elder, and the surgical operation patients so as to acquire a physiological signal of the user and to prevent undesired accidents of the limb-disabled persons, the elder, and the surgical operation patients.

Based on the above detailed description, those skilled in the art may appreciate that the present invention can achieve the above-discussed objectives. However, it is noted that the above description is made only to a preferred embodiment of the present invention and is not intending to limit the true scope where the present invention may be put into practice. Thus, simple and equivalent variations and modifications made on the disclosure of the specification and the attached claims are all considered within the scope of the present invention.

## Claims

1. A structure of a detective garment, comprising a garment body and at least one detection module combined with the garment body, the garment body comprising at least one opening section formed in a portion thereof.

2. The structure of the detective garment as claimed in Claim 1, wherein the garment body is a single-shoulder type garment and the opening section of the garment body comprises a fastening section.

3. The structure of the detective garment as claimed in Claim 1, wherein the garment body is a dual-shoulder garment and the opening section of the garment body is arranged at a shoulder and a lateral side of the garment, the opening section comprising a fastening section for each of the shoulder and the lateral side.

4. The structure of the detective garment as claimed in Claim 2, wherein the fastening section comprises a zipper.

5. The structure of the detective garment as claimed in Claim 3, wherein the fastening section comprises a zipper.

6. The structure of the detective garment as claimed in Claim 2, wherein the fastening section comprises hook-and-loop fasteners.

7. The structure of the detective garment as claimed in Claim 3, wherein the fastening section comprises hook-and-loop fasteners.

8. The structure of the detective garment as claimed in Claim 2, wherein the fastening section comprises a button assembly, the button assembly being arranged as one of single snap/button, dual snaps/buttons, and arrayed snaps/buttons.

9. The structure of the detective garment as claimed in Claim 3, wherein the fastening section comprises a button assembly, the button assembly being arranged as one of single snap/button, dual snaps/buttons, and arrayed snaps/buttons.

10. The structure of the detective garment as claimed in Claim 2, wherein the fastening section comprises a hook assembly, the hook assembly being arranged as one of single hook, dual hooks, and arrayed hooks.

11. The structure of the detective garment as claimed in Claim 3, wherein the fastening section comprises a hook assembly, the hook assembly being arranged as one of single hook, dual hooks, and arrayed hooks.

12. The structure of the detective garment as claimed in Claim 1, wherein the detection module comprises an electrode plate that is adapted to detect a physiological signal of a surface layer of a human body and the physiological signal is one of body temperature, heartbeat, and pulse.

13. The structure of the detective garment as claimed in Claim 1, wherein the detection module comprises an inclination detection chip and a microcontroller, the microcontroller being connected to the inclination detection chip so that the microcontroller detects a variation of the inclination detection chip.
